# EUROPEAN PATENT SPECIFICATION

(11) **EP 0 930 300 B1**
(45) Date of publication and mention of the grant of the patent: **22.01.2003**
(21) Application number: 98123861.1
(22) Date of filing: 16.12.1998
(51) Int. Cl.: C07D 213/61

(54) **Process for preparing (poly)chloropyridines**
Verfahren zur Herstellung von (Poly)ChloroPyridine
Procédé pour la préparation de (poly)chloropyridines

(30) Priority: 16.12.1997 JP 34616897
(43) Date of publication of application: 21.07.1999
(73) Proprietor: DAICEL CHEMICAL INDUSTRIES, Ltd., Sakai-shi, Osaka 590-0905 (JP)
(72) Inventor: Ikura, Kiyoshi, Arai-shi, Niigata 944-0018 (JP); Katsumata, Nobuhiro, Arai-shi, Niigata 944-0041 (JP)
(74) Representative: Kovacs, Paul

(56) References cited:
- WO-A-98/50362
- GB-A- 1 215 387
- US-A- 4 127 575
- US-A- 4 172 203
- US-A- 4 515 953
- PATENT ABSTRACTS OF JAPAN vol. 15, no. 472 (C-889), 29 November 1991 & JP 03 200769 A (TAJIKA HARUMI), 2 September 1991
- DATABASE WPI Week 944 Derwent Publications Ltd., London, GB; AN 31786 XP002095336 & JP 05 339235 A (DAICEL CHEM IND LTD) , 21 December 1993

## Description

### FIELD OF THE INVENTION

The present invention relates to a process for preparing pyridine chloride which is useful as an intermediate for pesticide and drug.

### DESCRIPTION OF THE RELATED ART

Heretofore, various processes for preparing pyridine chlorine have been suggested.

For example, Japanese Patent Kokai Publication No. 249965/1986 discloses a process for preparing pyridine chloride, which comprises applying a liquid phase chlorination method to pyridine or pyridine hydrochloride. However, according to this process, various pyridine chlorides are formed and, therefore, it is difficult to isolate specific pyridine chloride and the isolation step becomes complicated.

Japanese Patent Kokai Publication No. 206564/1983 discloses a process for preparing pyridine chloride, which comprises applying a gas phase chlorination method to chlorinated pyridine. However, since this process causes a gas phase reaction, the reaction step is complicated and, moreover, various pyridine chlorides are formed like the liquid phase chlorination method described above.

Great Britain Patent No. 1215387 discloses a process for preparing 2,3,5-trichloropyridine, which comprises chlorination of 2-amino-3,5-dichloropyridine as a starting material. However, according to this process, starting material is expensive and, therefore, this process is disadvantageous in view of the manufacturing cost.

U.S. Patent No. 4,127,575 discloses a process for preparing 2,3,5-trichloropyridine, which comprises oxidizing 2,3,5-trichloro-6-hydrazinopyridine or 2,3,5-trichloro-4-hydrazinopyridine with sodium hypochlorite in a solvent. This process is superior in selectivity of the desired product. However, according to this process, since a large amount of a solvent such as toluene must be used and, moreover, sodium hypochlorite must be supplied in the form of an aqueous solution having a concentration of about 15%, so-called space-time yield is very low. Moreover, according to this process, a large amount of sodium hypochlorite having a high unit cost must be used. In addition, since a cheap reaction vessel made of a stainless steel is corroded by sodium hypochlorite, an expensive reaction vessel must be used. Therefore, the manufacturing cost is increased.

Japanese Patent Publication JP 5-339 235 refers to oxidation reaction of chlorohydrazinopyridines to obtain dehalogenated pyridine derivatives. According to the process disclosed in this document, halopyridine and hydrazine are reacted together in alcohols having three or more carbon atoms. Water is then added to the reaction mixture and an organic layer is separated. In a following step, water is added to the thus obtained organic layer and alcohol is removed therefrom, by distillation, so as to obtain a liquid mixture of hydrazinopyridine and water. This mixture is then reacted with an oxidation reagent so as to obtain pyridine derivative(s). This process does not involve the use of a phase transfer catalyst and its manufacturing cost is not particularly advantageous.

As a process for preparing pyridine chloride to solve these disadvantages, Japanese Patent Kokai Publication No. 200769/1991 discloses a process for preparing pyridine chloride, which comprises oxidizing 2,3,5-trichloro-6-hydradinopyridine, 3,6-dichloro-2-hydrazinopyridine or 3,5-dichloro-2-hydrazinopyridine with hydrogen peroxide in a solvent and extracting with an organic solvent, followed by concentration and fractionation. By employing this process, the yield of pyridine chloride can be enhanced to some extent, thereby making it possible to reduce the manufacturing cost.

However, when this process for preparing pyridine chloride, comprising oxidizing with hydrogen hypochlorite is scaled up to an industrial level, separation between water and an organic solvent becomes poor. The cause of this poor separation is not known in detail, but is considered that poor separation is caused by the formation of tar as byproducts. There is a problem that the yield of pyridine chloride is lowered by this poor separation. There is also a problem that the production step becomes complicated by applying a countermeasure to the poor separation and, therefore, it becomes impossible to sufficiently reduce the manufacturing cost of pyridine chloride. Accordingly, a development of a process capable of preparing pyridine chloride in a higher yield at a lower cost in an industrial production step is required at present.

### SUMMARY OF THE INVENTION

The present invention has been accomplished to solve these problems, and an object of the present invention is to provide a process capable of preparing pyridine chloride in a high yield at a low cost.

The invention accomplished to solve the above problems provides a process for preparing pyridine chloride, which comprises the reaction step of oxidizing chlorohydrazinopyridine represented by the following general formula (1): 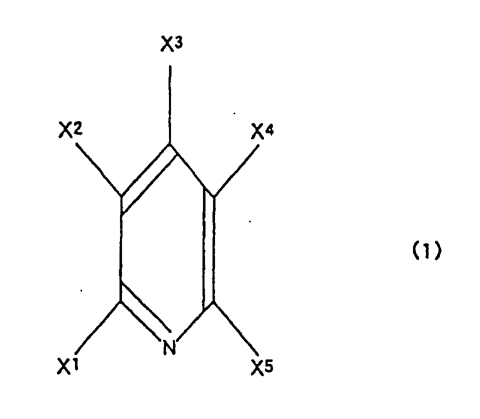 wherein X¹ to X⁵ are the same or different and represent a chlorine atom, a hydrogen atom or a hydrazino group (-NHNH₂), provided that at least one of X¹ to X⁵ represents a chlorine atom and at least one of X¹ to X⁵ represents a hydrazino group as a starting material, with hydrogen peroxide, characterized in that the reaction step of oxidizing said chlorohydrazinopyridine is conducted at a termperature of from 0 to 200°C, in the presence of a quaternary ammonium salt, as a phase transfer catalyst in a solvent mixture of water and an organic solvent which is insoluble, practically insoluble or slightly soluble in water, and in the presence of a basic substance, in an amount of 1 to 1000 parts by weight, based on 100 parts by weight of chlorohydrazinopyridine.

According to the present invention, since chlorohydrazinopyridine is oxidized with hydrogen peroxide in the presence of a phase transfer catalyst in a mixed solvent of an organic solvent and water, poor separation between water and the organic solvent does not occur after the completion of the reaction. Accordingly, pyridine chloride can be obtained in a high yield. Moreover, since a countermeasure to the poor separation, which makes the producing step complicated, is not required, the manufacturing cost of pyridine chloride can be reduced.

In the present invention, a mixing ratio of the organic solvent to water in the solvent is preferably from 1/100 to 100/1 in term of a weight ratio, and the amount of the solvent used is preferably from 10 to 1000 parts by weight based on 100 parts by weight of chlorohydrazinopyridine. Consequently, the separation property after the formation of pyridine chloride can be improved, thereby making it possible to reduce the production cost of pyridine chloride.

Furthermore, in the present invention, the amount of the phase transfer catalyst used is preferably from 0.01 to 100 parts by weight based on 100 parts by weight of chlorohydrazinopyridine. Consequently, the yield of pyridine chloride can be further reduced.

In these inventions, if a basic substance is added in the reaction system in the reaction step, the reaction for formation of pyridine chloride is promoted, thereby making it possible to obtain pyridine chloride in a high efficiency.

These inventions are particularly effective when trichloropyridine out of pyridine chloride is prepared by using trichlorohydrazinopyridine

### DESCRIPTION OF THE PREFERRED EMBODIMENTS

In the process of the present invention, chlorohydrazinopyridine represented by the above chemical formula (1) is used as a starting material and this chlorohydrazinopyridine is oxidized with hydrogen peroxide to substitute a hydrazino group for a hydrogen atom, thereby obtaining pyridine chloride. When chlorohydrazinopyridine as a starting material is monochlorohydrazinopyridine, monochloropyridine can be obtained. When chlorohydrazinopyridine as a starting material is dichlorohydrazinopyridine, dichloropyridine can be obtained. When it is trichlorohydrazinopyridine, trichloropyridine can be obtained. When it is tetrachlorohydrazinopyridine, tetrachloropyridine can be obtained. Thus, specific pyridine chloride can be formed by selecting the starting material and the selectivity of the product can be enhanced.

Chlorohydrazinopyridine as the starting material can be obtained by a known method. Chlorohydrazinopyridine can be obtained, for example, by selectively substituting a chlorine atom situated in the predetermined position of existing pyridine chloride for a hydrazino group. The pyridine chloride obtained by substituting a hydrazino group of this chlorohydrazinopyridine for a hydrogen atom corresponds to those obtained by selectively substituting a chlorine atom situated at the predetermined position of existing pyridine chloride for a hydrogen atom. Therefore, the number of chlorine atoms of the resulting pyridine chloride is smaller than that of existing pyridine chloride. For example, tetrachloropyridine can be obtained from pentachloropyridine via tetrachlorohydrazinopyridine, trichloropyridine can be obtained from tetrachloropyridine via trichlorohydrazinopyridine, and dichloropyridine can be obtained from trichloropyridine via dichlorohydrazinopyridine. In such way, the process for preparing chlorohydrazinopyridine by selectively substituting a chlorine atom situated at the position of existing pyridine chloride for a hydrazino group includes, for example, a process of reacting existing pyridine chloride with hydrazine hydrate, particularly a process of controlling the equivalent number of hydrazine hydrate (see Japanese Patent Kokai Publication No. 339235/1993; J. Chem. Soc., (c) 167, published in 1971).

The oxidizing reaction using hydrogen peroxide is conducted in a solvent mixture obtained by mixing an organic solvent with water, thereby making it possible to prevent poor separation after the completion of the pyridine chloride formation reaction. As the organic solvent, an organic solvent, which is insoluble, practically insoluble or slightly soluble in water, is used. The organic solvent used suitably in the present invention includes, for example, pentane, hexane, heptane, octane, benzene, toluene, xylene, mesitylene and ethylbenzene.

A mixing ratio of the organic solvent to water in the solvent mixture is preferably from 1/100 to 100/1, and particularly from 1/10 to 10/1. When the mixing ratio is smaller than the above range, the yield of pyridine chloride is lowered sometimes. On the contrary, when the mixing ratio exceeds the above range, the reaction rate is decreased sometimes and the yield of pyridine chloride is lowered sometimes.

The amount of the solvent used is preferably not less than 10 parts by weight, and particularly not less than 50 parts by weight, based on 100 parts by weight of chlorohydrazinopyridine. When the amount of the solvent is smaller than the above range, the yield of pyridine chloride is lowered sometimes. No problems arise even if the amount of the solvent is large. However, the amount is preferably not more than 1000 parts by weight, and practically not more than 500 parts by weight, based on 100 parts by weight chlorohydrazinopyridine, taking the prevention of a reduction in space-time yield into consideration.

In the present invention, chlorohydrazinopyridine is oxidized with hydrogen peroxide in the presence of a phase transfer catalyst, thereby making it possible to increase the yield of pyridine chloride. The term "phase transfer catalyst" used herein means all substance, which promote transfer of ions, charged molecules and neutral molecules existing in each phase to the other phase in a solvent forming two phases which are heterogeneous each other, and includes, for example, quaternary ammonium salt, phosphonium salt, amine, crown ether and cryptand.

The quaternary ammonium salt, used as a phase transfer catalyst in the present invention includes, for example, tetraethylammonium chloride, tetraethylammonium bromide, tetrabutylammonium chloride, tetrabutylammonium hydrogen sulfate, tetrabutylammonium bromide, tetrabutylammonium iodide, tetrabutylammonium hydroxide, tetraoctylammonium chloride, tributylbenzylammonium chloride, tributylbenzylammonium bromide, trioctylbenzyl ammonium chloride, benzyltriethylammonium chloride, cetyldimethylbenzylammonium chloride, tributylbenzyl ammonium chloride and benzyltrimethyl ammonium chloride. The phase transfer catalyst used particularly suitably includes benzyltriethylammonium chloride and cetyldimethylbenzylammonium chloride.

The amount of the quaternary ammonium salt used as a phase transfer catalyst is preferably not less than 0.01 parts by weight, and particularly not less than 0.1 parts by weight, based on 100 parts by weight of chlorohydrazinopyridine. When the amount of the phase transfer catalyst is smaller than the above range, the reaction rate is lowered sometimes and the yield of pyridine chloride is lowered sometimes. No problems arise even if the amount of the phase transfer catalyst is large. However, the amount is preferably not more than 100 parts by weight, and practically not more than 10 parts by weight, based on 100 parts by weight chlorohydrazinopyridine, taking the prevention of an increase in manufacturing cost into consideration.

In the present invention, a basic substance is added in the reaction system. By adding the basic substance, hydrazine hydrate is activated, thereby enhancing the formation efficiency of pyridine chloride. The basic substance to be added may be an inorganic or organic base, but is preferably inorganic base because the purification step is simplified. Preferred inorganic base include, for example, sodium hydrogencarbonate, sodium hydroxide, potassium hydroxide, sodium carbonate, potassium carbonate and potassium hydrogencarbonate. Among them, sodium hydrogencarbonate is particularly preferred.

The amount of the basic substance added varies depending on the amount of an aqueous solvent, but is preferably from 1 to 1000 parts by weight, and particularly from 50 to 200 parts by weight, based on 100 parts by weight of chlorohydrazinopyridine. When the amount of the basic substance is smaller than the above range, the yield of pyridine chloride is lowered sometimes. To the contrary, when the amount of the basic substance exceeds the above range, the purification step becomes complicated sometimes.

It is necessary that the molar amount of hydrogen peroxide used in the present invention is the same as or larger than that of chlorohydrozinopyridine, and 1-3 equimolar amount of hydrogen peroxide is usually used. The oxidizing reaction using hydrogen peroxide is usually conducted over 1 to 20 hours. The reaction is conducted at 0 to 200°C, and preferably at 50 to 100°C. Hydrogen peroxide is charged in the reaction system in the form of aqueous hydrogen peroxide. Aqueous hydrogen peroxide may also be charged at a time, but is usually added dropwise over about 1 to 15 hours.

After the completion of the reaction, the organic layer is separated and this organic layer is concentrated by fractional distillation to obtain pyridine chloride.

### EXAMPLE

The following Examples further illustrate the present invention in detail.

### Example 1

50 kg of 2,3,5-trichloro-6-hydrazinopyridine as a starting material, 45 kg of toluene as an organic solvent, 100 kg of water, 50 kg of sodium hydrogencarbonate as a basic substance and 3 kg of benzyltriethylammonium chloride as a phase transfer catalyst were charged in order in a reaction vessel. The reaction system was heated to 75 and 27 kg of aqueous 35% hydrogen peroxide was added dropwise in the reaction vessel over 10 hours.

After the completion of the reaction, the reaction solution was allowed to stand for about 30 minutes and then observed visually. As a result, the interface between the organic layer and aqueous layer was clear and the separation property was good. This organic layer was concentrated by fractional distillation to obtain 2,3,5-trichloropyridine. The yield of 2,3,5-trichloropyridine was calculated based on 2,3,5-trichloro-6-hydrazinopyridine. As a result, the yield was 93%.

### Example 2

According to the same manner as that described in Example 1 except for adding 3 kg of cetyldimethylbenzylammonium chloride in place of benzyltriethylammonium chloride as the phase transfer catalyst, the oxidizing reaction using hydrogen oxide was conducted.

After the completion of the reaction, the reaction solution was allowed to stand for about 30 minutes and then observed visually. As a result, the interface between the organic layer and aqueous layer was clear and the separation property was good. This organic layer was concentrated by fractional distillation to obtain 2,3,5-trichloropyridine. The yield of 2,3,5-trichloropyridine was calculated based on 2,3,5-trichloro-6-hydrazinopyridine. As a result, the yield was 95%.

### Comparative Example 1

50 kg of 2,3,5-trichloro-6-hydrazinopyridine as a starting material, 100 kg of water, 65 kg of an aqueous 25% sodium hydroxide solution as a basic substance were charged in order in a reaction vessel. The reaction system was heated to 75°C and 27 kg of aqueous 35% hydrogen peroxide was added dropwise in the reaction vessel over 10 hours. After the completion of the reaction, 45 kg of toluene was charged.

The reaction solution was allowed to stand for about 120 minutes and then observed visually. As a result, a scum-like substance is present at the interface between the organic layer and aqueous layer in the state of being surrounded by both layers and, therefore, the separation property was poor. This organic layer was concentrated by fractional distillation to obtain 2,3,5-trichloropyridine. The yield of 2,3,5-trichloropyridine was calculated based on 2,3,5-trichloro-6-hydrazinopyridine. As a result, the yield was 84%.

### Comparative Example 2

According to the same manner as that described in Example 1 except for adding no benzyltriethylammonium chloride, the oxidizing reaction using hydrogen oxide was conducted.

The reaction solution was allowed to stand for about 120 minutes and then observed visually. As a result, a scum-like substance is present at the interface between the organic layer and aqueous layer in the state of being surrounded by both layers and, therefore, the separation property was poor. This organic layer was concentrated by fractional distillation to obtain 2,3,5-trichloropyridine. The yield of 2,3,5-trichloropyridine was calculated based on 2,3,5-trichloro-6-hydrazinopyridine. As a result, the yield was 23%.

The above results are shown in Table 1 below.

| | Example 1 | Example 2 | Comp. Example 1 | Comp. Example 1 |
|---|---|---|---|---|
| Solvent | Toluene/water | Toluene/Water | Water | Toluene/Water |
| Phase transfer catalyst | Benzyltriethylammonium chloide | Cetyldimethylbenzylammonium chloride | - | - |
| Basic substance | Sodium hydrogencarbonate | Sodium hydrogencarbonate | Sodium hydroxide | Sodium hydrogencarbonate |
| Separation property | Good | Good | Poor | Poor |
| Yield (%) | 93 | 95 | 84 | 23 |

As is apparent from Table 1, the processes of Example 1 and Example 2, wherein chlorohydrazinopyridine is oxidized in the presence of a phase transfer catalyst in a mixed solvent of an organic solvent and water, is superior in separation property and yield to the process of Comparative Example 1 using no phase transfer catalyst in a solvent of water alone and the process of Comparative Example 2 using no phase transfer catalyst. The superiority of the process of the present invention was proved by these test results.

As described above, the present invention provides a process for preparing pyridine chloride, which can prepare the desired product in a high yield and requires no countermeasure for poor separation because of good separation property, thereby making it possible to obtain the desired product at a low cost.

Since this process is superior in selectivity of the desired product, various pyridine chlorides are not formed. Therefore, it is not necessary to provide the step of isolating specific pyridine chloride.

## Claims

1. A process for preparing pyridine chloride, which comprises the reaction step of oxidizing chlorohydrazinopyridine represented by the following general formula (1): 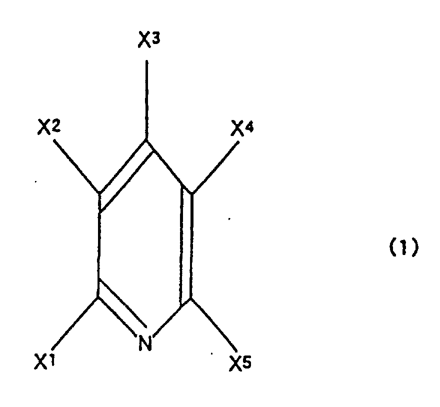 wherein X¹ to X⁵ are the same or different and represent a chlorine atom, a hydrogen atom or a hydrazino group (-NHNH₂), provided that at least one of X¹ to X⁵ represents a chlorine atom and at least one of X¹ to X⁵ represents a hydrazino group as a starting material, with hydrogen peroxide, **characterized in that** the reaction step of oxidizing said chlorohydrazinopyridine is conducted at a temperature of from 0 to 200°C, in the presence of a quaternary ammonium salt, as a phase transfer catalyst in a solvent mixture of water and an organic solvent which is insoluble, practically insoluble or slightly soluble in water, and in the presence of a basic substance, in an amount of 1 to 1000 parts by weight, based on 100 parts by weight of chlorohydrazinopyridine.

2. The process for preparing pyridine chloride according to claim 1, wherein the mixing ratio of the organic solvent to water in said solvent mixture is from 1/100 to 100/1 in terms of weight ratio.

3. The process for preparing pyridine chloride according to claim 1, wherein the amount of the solvent used is from 10 to 1000 parts by weight based on 100 parts by weight of chlorohydrazinopyridine.

4. The process for preparing pyridine chloride according to claim 1, wherein the amount of the quaternary ammonium salt is from 0.01 to 100 parts by weight based on 100 parts by weight of chlorohydrazinopyridine.

5. The process for preparing pyridine chloride according to any one of claims 1 through 4, wherein chlorohydrazinopyridine of the starting material is trichlorohydrazinopyridine and the resulting pyridine chloride is trichloropyridine.

## Patentansprüche

1. Verfahren zur Herstellung von Pyridinchlorid umfassend den Reaktionsschritt des Oxydierens von einem Chlorhydrazinopyridin der allgemeinen Formel (1) : 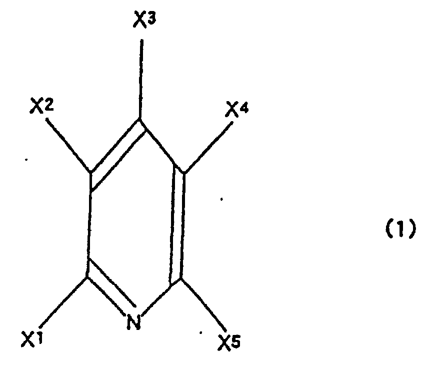 worin X¹ bis X⁵ gleich oder verschieden sind und ein Chloratom, ein Wasserstoffatom oder eine Hydrazinogruppe (-NHNH₂) darstellen, unter der Bedingung, dass wenigstens eine der Gruppe X¹ bis X⁵ ein Chloratom darstellt und wenigstens eine der Gruppe X¹ bis X⁵ eine Hyrazinogruppe darstellt, als Ausgangsstoff mit Wasserstoffperoxid, **dadurch gekennzeichnet, dass** den Reaktkonsschritt des Oxydierens des oben erwähnten Chlorhydrazinopyridins bei einer Temperatur von 0 bis 200°C geführt wird, in Gegenwart von einem quaternären Ammoniumsalz, als Phasenversetzungskatalysator, in einer Lösungsmittelmischung von Wasser und einem organischen Lösungsmittel, das unlöslich, praktisch unlöslich oder schachlöslich im Wasser ist, und in Gegenwart von einem basischen Stoff, in einer Menge von 1 bis 1000 Gewichtsanteilen, auf der Basis von 100 Gewichtsanteilen von Chlorhydrazinopyridin.

2. Verfahren zur Herstellung von Pyridinchlorid nach Anspruch 1, worin das Mischungsverhältnis des organischen Lösungsmittels zum Wasser, in der genannten Lösungsmittelsmischung, 1/100 bis 100/1 als Gewichtsanteilsverhältnis beträgt.

3. Verfahren zur Herstellung von Pyridinchlorid nach Anspruch 1, worin die Menge des verbrauchten Lösungsmittels 10 bis 1000 Gewichtsanteile von Chlorhydroazinopyridin beträgt.

4. Verfahren zur Herstellung von Pyridinchlorid nach Anspruch 1, worin die Menge des quaternären Ammoniumsalzes 0.01 bis 100 Gewichtsanteilen auf der Basis von 100 Gewichtsanteilen von Chlorhydrazinopyridin beträgt.

5. Verfahren zur Herstellung von Pyridinchlorid nach einem der Ansprüche 1 bis 4, worin der Ausgangsstoff Chlorhydryzinoyridin aus Trichlorhydrazinopyridin, besteht und das entstehende Pyridinchlorid aus Trichlorpyridin besteht.

## Revendications

1. Procédé de préparation de chloropyridine, comprenant l'étape d'oxydation de chlorohydrazinopyridine, représentée par la formule générale suivante (1). 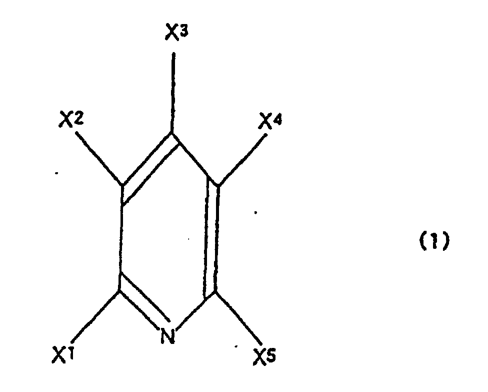 dans laquelle X¹ à X⁵ sont identiques ou différents et représentent un atome de chlore, un atome d'hydrogène ou un groupe hydrazino (-NHNH₂), à la condition que au moins l'un des symboles X¹ à X⁵ représente un atome de chlore et au moins l'un des symboles X¹ à X⁵ représente un groupe hydrazino, en tant que substance de départ, par l'eau oxygénée, **caractérisé en ce que** l'étape d'oxydation de ladite chlorohydrazinopyridine est effectuée à une température de 0 à 200°C, en présence d'un sel d'ammonium quaternaire, en tant que catalyseur de transfert de phase, dans un mélange solvant constitué d'eau et d'un solvant organique, insoluble, pratiquement insoluble ou faiblement soluble dans l'eau, et en présence d'une substance basique, en une quantité de 1 à 1000 parties, en poids, pour 100 parties, en poids de chlorohydrazinopyridine.

2. Procédé de préparation de chloropyridine, selon la revendication 1, selon lequel la proportion pondérale de solvant organique par rapport à l'eau, dans ledit mélange solvant, est de 1/100 à 100/1.

3. Procédé de préparation de chloropyridine, selon la revendication 1, selon lequel la quantité de solvant utilisée est de 10 à 1000 parties, en poids, pour 100 parties, en poids, de chlorohydrazinopyridine.

4. Procédé de préparation de chloropyridine, selon la revendication 1, selon lequel la quantité de sel d'ammonium quaternaire est de 0,01 à 1000 parties, en poids, pour 100 parties, en poids, de chlorohydrazinopyridine.

5. Procédé de préparation de chloropyridine, selon l'une des revendications 1 à 4, dans lequel la chlorohydrazinopyridine, constituant le substance de départ, est la trichlorohydrazinopyridine et la chloropyridine résultante est la trichloropyridine.
